# EUROPEAN PATENT APPLICATION

(11) **EP 4 442 238 A1**
(43) Date of publication of application: **09.10.2024**
(21) Application number: 23166820.3
(22) Date of filing: 05.04.2023
(51) Int. Cl.: A61K 6/62, A61K 6/78, A61K 6/887, A61K 6/76, A61K 6/77

(54) **A CURABLE DENTAL COMPOSITION COMPRISING TOCOPHEROL, ITS DERIVATIVES AND ISOMERS AS A POLYMERIZATION INHIBITOR**

(71) Applicant: DENTSPLY SIRONA Inc., York, PA 17401 (US); DENTSPLY DETREY GmbH, 78467 Konstanz (DE)
(72) Inventor: TRÖTSCHLER, Tobias, 78467 KONSTANZ (DE); FREDRICH, Sebastian, 78467 KONSTANZ (DE)
(74) Representative: Pichova, Vanda

(57) **Abstract**

A curable dental composition comprising a monomer mixture and optionally at last one pigment, wherein the monomer mixture comprises at least one polymerizable monomer, at least one polymerization initiator and at least one polymerization inhibitor, wherein the at least one polymerization inhibitor is Tocopherol, its derivative, ether or ester or one of their isomers or a mixture thereof. The curable dental composition is preferably a light curable dental composition comprising at least one photoinitiator. The curable dental composition comprises preferably at least one filler.

## Description

### BACKGROUND OF THE INVENTION

Dental compositions face the problem that monomers used undesirably spontaneously react during various stages of the manufacture, processing, handling, storage, and use. It is well known that the monomers readily polymerize during storage and that such polymerization increases when temperature increases. Utilization of monomers in the temporary and permanent dental restorations such as composites, adhesives, cements, and dentures requires stability of the monomer mixtures until polymerization is desired to ensue. To reduce the chance of uncontrolled initiation of polymerization, the monomers are usually treated with polymerization inhibitors, such as hydroquinone (HQ), methyl ether of hydroquinone (MeHQ), butylated hydroxytoluene (BHT), benzoquinone, tert-butyl catechol.

All of the above polymerization inhibitors show smaller or larger degree of biocompatibility issues. BHT is under assessment as an endocrine disruptor. MeHQ is skin sensitizing and harmful to aquatic life with long lasting effects. HQ is suspected to be carcinogenic and mutagenic. Tert-butyl catechol causes severe skin burns, allergic reactions, eye damage and it is very toxic to aquatic life with long lasting effect.

It is an object of the present invention to overcome the above shortcomings.

U.S. Patent No. 5,461,124 discloses the use of vitamin E as an inhibitor against premature polymerization in reactive systems capable of undergoing a free radical initiated polymerization which system before and/or after polymerization thereof are placed in tissue contact with a living body. The patent is dealing with methyl methacrylate and poly(methylmethacrylate) of bone cements. The reactive composition of the patent is monocomponent or multicomponent medical and/or dental -medical adhesives, cements, or fillers based on conventional monofunctional, and/or polyfunctional olefinically unsaturated compounds, particularly acrylic acid and or methacrylic acids or derivatives thereof.

U.S.Pat. No. 6,106,820 describes cosmetic compositions that include nail polish that can optionally contain vitamins. Disclosed are cosmetic compositions comprising: (a) from about 0.1% to about 50%, based on the weight of the composition, of a film-forming graft copolymer, wherein the copolymer comprises: (i) a backbone exhibiting a Tg of from about 0 DEG C. to about 50 DEG C.; and (ii) one or more hydrophilic grafts attached to the backbone wherein each of the grafts exhibits a Tg of from about 50 DEG C. to about 200 DEG C., and wherein the average molecular weight of each of the grafts is greater than about 1000; and (b) from about 50% to about 99.9%, based on the weight of the composition, of a suitable carrier. The compositions provide good wear properties and are suitable for use in a variety of cosmetic applications, such as facial moisturizers, foundations, lipsticks, mascaras, nail polishes and the like. In a preferred embodiment, the compositions are in the form of a nail polish which, when applied to nails, exhibit excellent long wear properties. The compositions may include Tocopherol or Tocopherol acetate.

U.S. Patent application 2003/0191338 discloses Tocopherol as an inhibitor for thermally cured methacryl-based resins in dental materials. It does not, however, cover filled compositions and/or light cured compositions. It does not disclose use of various isomers and/or esters of Tocopherol either.

### BRIEF SUMMARY

It is an object of the present invention to introduce an environmentally friendly and biocompatible curable dental composition.

It is an object of the present invention to introduce Tocopherol, its derivative, ether or ester or one of their isomers or a mixture thereof as a polymerization inhibitor of a curable dental composition. The curable dental composition of the present invention is preferably a dental composition comprising a filler.

The curable dental composition is preferably a light curable dental composition.

The curable dental composition is preferably a light curable dental composition comprising at least one photoinitiator.

In another embodiment of the present invention, the curable dental composition comprises a redox system as an initiator.

The embodiments described herein use Tocopherol, its derivative, ether or ester or one of their isomers or a mixture thereof as a polymerization inhibitor of the above curable dental compositions.

The isomers include, but are not limited to alpha, beta, gamma and delta-stereoisomers.

The Tocopherol esters and derivatives include, but are not limited to a Tocopherol acetate, a Tocopherol succinate and a Tocopherol carbamate.

**The following abbreviations and terms are used in the current specification:**
UDMA= Urethane dimethacrylate resin
DMABE = N,N-Dimethylamino ethyl benzoate
DPI = Dimethyl diphenyl iodonium hexafluorophosphate
EBPADMA = Ethoxylated bisphenol A dimethacrylate
BAABE = N,N'-diallyl-1,4- bisacrylamido-(2E)-but-2-ene
BADEP = N,N'-diethyl-1,3-bisacrylamido-propane
CQ = Camphorquinone
Spectrum TPH = Spectrum Total Performance Hybrid
BHT = Butylated hydroxytoluene
Paste = A curable dental composition comprising a filler
TEGDMA = Triethylenglycol dimethacrylate
BisEMA = [2-ethoxy-3-[4-[2-[4-[2-ethoxy-3-(2-methylprop-2-enoyloxy)propoxy]phenyl]propan-2-yl]phenoxy]propyl] 2-methylprop-2-enoate
Tocopherol = Vitamin E

Isomers in the sense of this invention are stereoisomers, including enantiomers.

Inhibitor in the sense of this invention is a polymerization inhibitor.

### DESCRIPTION OF THE FIGURES

Objects, features, and advantages of the present invention will also become apparent upon reading the following description in conjunction with the figures and tables.

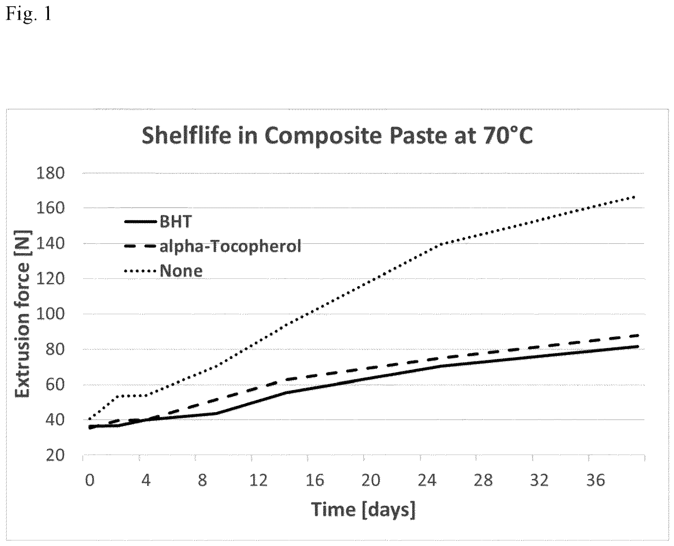

**Fig. 1** depicts extrusion forces of pastes comprising BHT, alpha-Tocopherol or no polymerization inhibitor at all, respectively, from compules comprising filler at 70°C over time indicating the behavior over a shelflife of about two years. It is an accelerated shelflife extrapolated to two years at room temperature. A higher extrusion force is obtained when the paste is partially cured. It is therefore a sign of polymerization and inefficient stabilization. The extrusion force of the paste without any inhibitor rapidly increases whereas pastes with BHT and alpha-Tocopherol behave in a similar way and only increase slowly in extrusion force. The inhibition efficiency of both polymerization inhibitors is equal.

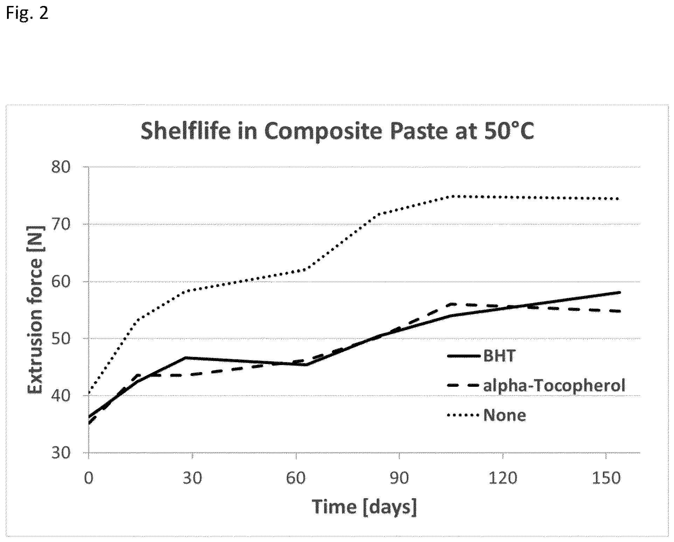

**Fig. 2** depicts extrusion forces of pastes comprising BHT, alpha-Tocopherol or no polymerization inhibitor at all, respectively, from compules comprising filler at 50°C over time indicating the behavior over a shelflife of about two years. It is an accelerated shelflife extrapolated to two years at room temperature. A higher extrusion force is obtained when the paste is partially cured. It is therefore a sign of polymerization and inefficient stabilization. The extrusion force of the paste without any inhibitor rapidly increases whereas pastes with BHT and alpha-Tocopherol behave similar and only increase slowly in extrusion force. The inhibition efficiency of both polymerization inhibitors is equal.

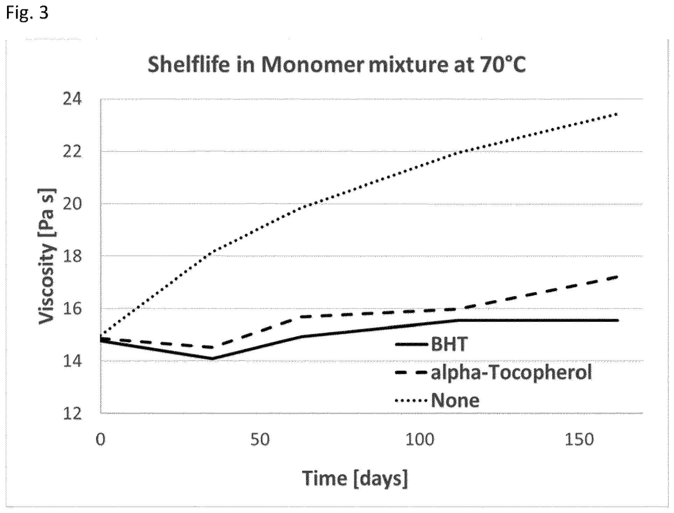

**Fig. 3** depicts viscosity of monomer mixtures comprising BHT, alpha-Tocopherol or no polymerization inhibitor at all, respectively, comprising no filler, at 70°C over time indicating the behavior over a shelflife of about two years. It is an accelerated shelflife extrapolated to two years at room temperature. A higher viscosity is obtained when the mixture is partially cured. It is therefore a sign of polymerization and inefficient stabilization. The viscosity of the liquid without any inhibitor rapidly increases whereas mixtures with BHT and alpha-Tocopherol behave similar and only increase slowly in viscosity. The inhibition efficiency of both polymerization inhibitors is equal.

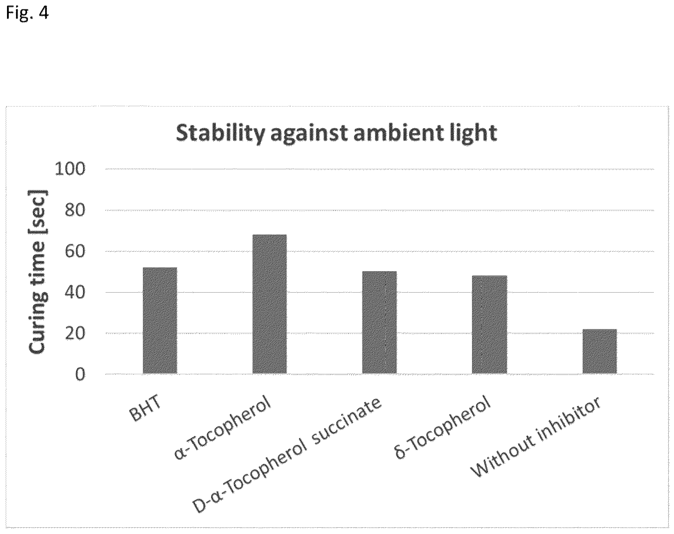

**Fig. 4** depicts light curing times of monomer mixtures containing 0.25 wt% CQ, 0.75 wt% DMABE, and 0.25wt% of different polymerization inhibitors, including BHT, isomers of Tocopherol and one resin not comprising any inhibitor. This time indicates the working time for the operator to treat the monomer mixture under ambient light before polymerization occurs. A longer time is obtained if the inhibitor suppresses the polymerization more effectively. All formulations differing just in the kind of inhibitor show similar working times and thus a similar inhibition efficiency of Tocopherol and BHT. Alpha - Tocopherol has a superior efficiency. Delta-Tocopherol and D-Alpha-Tocopherol succinate show a similar inhibition efficiency as BHT. All polymerization inhibitors perform better than the comparison without a polymerization inhibitor.

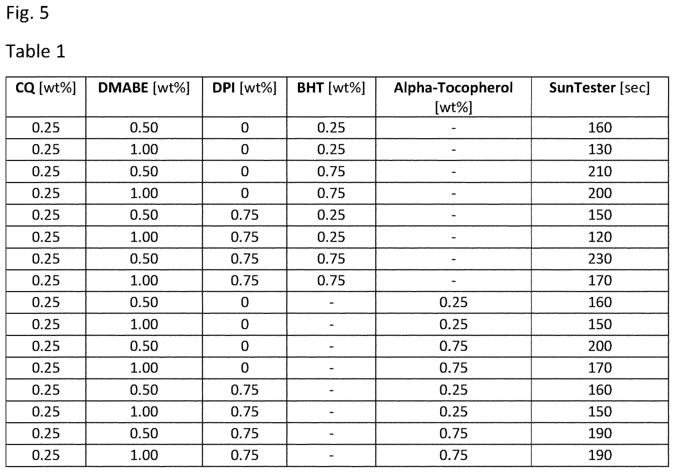

**Fig. 5** **(Table 1)** depicts composition of monomer mixtures comprising no filler and comprising UDMA, CQ, DMABE, DPI in varying ratios. They contain either BHT or alpha-Tocopherol as a polymerization inhibitor against ambient light. The light curing time (at 9000 Lux, broad band spectrum) in seconds is disclosed. This time indicates the working time for the operator to treat the monomer mixture under ambient light before polymerization occurs. A longer time is obtained if the inhibitor suppresses the polymerization more effectively. All formulations differing just in the kind of a polymerization inhibitor show similar working times and thus a similar inhibition efficiency of alpha-Tocopherol and BHT.

### DETAILED DESCRIPTION

Described herein is a curable dental composition comprising a monomer mixture and optionally at last one pigment, wherein the monomer mixture comprises at least one polymerizable monomer, at least one polymerization initiator and at least one polymerization inhibitor, wherein the at least one polymerization inhibitor is Tocopherol, its derivative, ether or ester or one of their isomers or a mixture thereof.

The curable dental composition is preferably a light curable dental composition. In that case the at least one polymerization initiator is at least one photoinitiator.

In another embodiment of the present invention, the curable dental composition comprises a redox system as a polymerization initiator.

The curable dental composition comprises preferably additionally at least one filler.

The curable dental composition may also comprise one or more compounds selected from a UV stabilizer, a rheological modifier, a fluorescent agent, an acidic component, a chain transfer agent, an X-ray opaquer, a silanization agent, a reactive glass and a solvent.

The curable dental composition is produced by standard processes of the field, wherein Tocopherol, its derivative, ether or ester or one of their isomers or a mixture thereof are used as polymerization inhibitors.

The at least one polymerizable monomer used in the embodiments is selected from the group comprising a monofunctional or oligofunctional or polyfunctional vinyl, or acrylate or a methacrylate including but not limited to methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate, hydroxypropyl acrylate, hydroxypropyl methacrylate, tetrahydrofurfuryl acrylate, tetrahydrofurfuryl methacrylate, glycidyl acrylate, glycidyl methacrylate, the diglycidyl methacrylate of bis-phenol A, glycerol mono-and di- acrylate, glycerol mono-and di- methacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate, neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethylhexamethylene dicarbamate, di-1-methyl-2-methacryloxyethyl-dimethylbenzene dicarbamate, di-1 -methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1 -chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane, 2,2'-bis[3(4-phenoxy)-2-hydroxypropane-1-acrylate]propane, N,N'-diallyl-1,4- bisacrylamido-(2E)-but-2-ene (BAABE) and N,N'-diethyl-1,3-bisacrylamido-propane (BADEP).

In the preferred embodiments of the present invention, the one or more polymerizable monomers are selected from the group comprising EBPADMA-Resin, UDMA-Resin, TEGDMA, BisEMA.

The at least one polymerization inhibitor used in the embodiments of the present invention is selected from the group comprising a derivative, ether or ester of Tocopherol or one of their isomers or a mixture thereof. The mode of action of the Tocopherol based polymerization inhibitor is that it causes avoidance of spontaneous and unintended polymerization. Tocopherol based polymerization inhibitor acts as a free radical scavenger. Free radical scavengers are similar to the secondary antioxidants used to inhibit thermal oxidation; they react with free radicals. Tocopherol is one of the aerobic polymerization inhibitors because it requires oxygen to develop its effect. In contrast, anaerobic polymerization inhibitors are those that do not require oxygen, such as stable radicals like TEMPO, phenothiazine and Galvinoxyl.

The Tocopherol based polymerization inhibitor also gives the curable dental composition sufficient stability against ambient light and thermal processes. In addition to this, the Tocopherol based polymerization inhibitor is more soluble and biocompatible when compared with BHT or other polymerization inhibitors of the state of the art. The Tocopherol based polymerization inhibitor is thus environmentally friendly.

The at least one polymerization initiator used in the embodiments of the present invention is preferably a photoinitiator capable of promoting polymerization of the polymerizable groups on exposure to light of a suitable wavelength and intensity. A visible light and an ultraviolet light photoinitiator are preferred. A suitable visible light and ultraviolet light photoinitiator includes an alpha-diketone (e.g., camphorquinone) with or without at least one additional hydrogen donor (such as sodium benzene sulfinate, amines, amino alcohols, DMABE, dimethylaminobenzonitrile, iodonium salt, DPI or germanium organyls).

In the preferred embodiments of the present invention, the at least one initiator is selected from the group comprising CQ, DMABE and DPI.

The at least one filler is selected from the group comprising a glass filler, a silanated glass filler, zirconium oxide, fluorides including strontium fluoride and ytterbium fluoride, an organic filler, a hybrid organic/inorganic filler and a combination thereof.

The at least one filler is preferably a glass filler or a hybrid organic/inorganic filler.

The glass filler of the present invention is preferably a silanated glass filler.

The surface of the glass filler of the present invention may be modified prior to the mixing step. Accordingly, the surface modifying agent contains a modifying compound capable of reacting with surface atoms of the filler, thereby forming a covalent bond between the surface atoms of the filler and the modifying compound. Additionally, the modifying compound may contain one or more polymerizable double bonds reactive in the crosslinking reaction after the filler is modified. The modifying agent may contain one or more modifying compounds. Preferably, the modifying compound provides a polymerizable ligand capable of crosslinking which may be a compound of one of the following formulae (I), (II) and (III), or a hydrolysis product thereof

XᵣR₃₋ᵣSiL (I)

XᵣR₂₋ᵣSiL'L" (II)

XᵣSiL'L"L‴ (III)

wherein
- X: represents a hydrolyzable group;
- R: represents an alkyl, cycloalkyl, cycloalkylalkyl, aralkyl or aryl group, L, L', L", and L'"
which may be the same or different represent independent from each other an organic group containing one or more polymerizable double bonds;
- r: is an integer of 1 to 3,
whereby the sum of X, R, L, L', L", and L'" is 4 for each of formula (I), (II), and (III).

Preferably, X is a halogen atom or OR¹ , wherein R¹ is an alkyl, cycloalkyl, cycloalkylalkyl, aralkyl or aryl group. More preferably, R or R¹ are independently an alkyl group.

In order to impart crosslinking capability to the organofunctional silicon compound, L, L', L", and L'" contain one or more polymerizable double bonds capable of taking part in a crosslinking reaction. In a preferred embodiment, L, L', L", and L'" may be selected from the group of allyl, (meth)acrylic ester groups, and (meth)acrylic amide groups.

An alkyl group may be straight-chain or branched C1-16 alkyl group, typically a C1-8 alkyl group. Examples for a C1-6 alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. A cycloalkyl group may be a C3-16 cycloalkyl group. Examples of the cycloalkyl group can include those having 3 to 14 carbon atoms, for example, cyclopropyl, cyclobutyl, cyclopentyl and cyclohexyl. A cycloalkylalkyl group can include those having 4 to 22 carbon atoms. Examples for a cycloalkylalkyl group can include a combination of a linear or branched alkyl group having 1 to 6 carbon atoms and a cycloalkyl group having 3 to 14 carbon atoms. Examples of the cycloalkylalkyl group can for example, include methylcyclopropyl, methylcyclobutyl, methylcyclopentyl, methylcyclohexyl, ethylcyclopropyl, ethylcyclobutyl, ethylcyclopentyl, ethylcyclohexyl, propylcyclopropyl, propylcyclobutyl, propylcyclopentyl, propylcyclohexyl. An aralkyl group may be a C7-26 aralkyl group, typically a combination of a linear or branched alkyl group having 1 to 6 carbon atoms and an aryl group having 6 to 10 carbon atoms. Specific examples of an aralkyl group are a benzyl group or a phenylethyl group. An aryl group can include aryl groups having 6 to 10 carbon atoms. Examples of the aryl group are phenyl and naphthyl.

The C1-6 alkyl group and the C3-14 cycloalkyl group may optionally be substituted by one or more members of the group selected from a C1-4 alkyl group, C1-4 alkoxy group, a phenyl group, and a hydroxy group. Examples for a C1-4 alkyl group can include linear or branched alkyl groups having 1 to 4 carbon atoms, for example, methyl, ethyl, n-propyl, isopropyl, n-butyl, isobutyl, sec-butyl, tert-butyl. Examples for an C1-4 alkoxy group can include linear or branched alkoxy groups having 1 to 4 carbon atoms, for example, methoxy, ethoxy, n-propoxy, isopropoxy, n-butoxy, isobutoxy, sec-butoxy, and tert-butoxy.

Aryl groups may contain 1 to 3 substituents. Examples of such substituents can include halogen atoms, C1-4 alkyl groups, C1-4 alkoxy groups, C1-4 alkylthio groups, C1-4 alkylsulfonyl groups, carboxyl group, C2-5 alkoxycarbonyl groups, and C1-4 alkylamino groups. Here, illustrative of the halogen atoms can be fluorine, chlorine, bromine and iodine. The C1-4 alkyl groups are, for example, methyl, ethyl, n-propyl, isopropyl and n butyl. Illustrative of the C1-4 alkoxy groups are, for example, methoxy, ethoxy and propoxy. Illustrative of the C1-4 alkylthio groups are, for example, methylthio, ethylthio and propylthio. Illustrative of the C1-4 alkylsulfonyl groups are, for example, methylsulfonyl, ethylsulfonyl and propylsulfonyl. Illustrative of the C2-5 alkoxycarbonyl groups can be those having alkoxy groups each of which contains 1 to 4 carbon atoms, for example, methoxycarbonyl, ethoxy carbonyl and propoxycarbonyl. Illustrative of the C1-8 alkylamino groups can be those having one or two alkyl groups each of which contains 1 to 4 carbon atoms, for example, methylamino, dimethylamino, ethyl amino and propylamino. The alkyl moieties in these substituents may be linear, branched or cyclic.

The at least one pigment is preferably selected from the group comprising iron oxide, titanium oxide, barium sulfate, manganese oxide, zinc oxide, bismuth oxide and organic fluorophores.

The curable dental composition is for use in the dental field, more particularly for use in producing a composite, a temporary sealing material, a dental cement, an adhesive or a fissure sealant.

In the preferred embodiment of the present invention, the curable dental composition comprises 15-99.5% by weight of the at least one polymerizable monomer, 0.1-3% by weight of the at least one polymerization initiator, 0.001-3.0% by weight of the at least one polymerization inhibitor and 0-85% by weight of the at least one filler.

In another preferred embodiment of the present invention, the curable dental composition comprises 0.005-1.0 % by weight of the at least one polymerization inhibitor or 0.01-0.75 % by weight of the at least one polymerization inhibitor.

### EXAMPLES

**Example 1** describes preparation of a curable dental composition and a curable dental composition comprising a filler and measurement of extrusion force.

**Example 2** describes preparation of a curable dental composition and measurement of viscosity.

**Example 3** describes preparation of a curable dental composition and measurement of stability against ambient light.

**Example 4** describes preparation of a curable dental composition comprising isomers of Tocopherol and its ester and measurement of stability against ambient light.

### Example 1: Extrusion force of a curable dental composition comprising a filler

Three mixtures comprising 98 wt% EBPADMA, 1 wt% DMABE, 0.5 wt% camphorquinone and either inhibitor of BHT, alpha-Tocopherol or none (0.5 wt%, in the latter case replaced by 0.5 wt% more EBPADMA) were prepared and stirred for one hour in the dark at 50°C until all compounds are homogeneously mixed.

**Table 2. Composition of monomer mixture A containing BHT.**

| Compound | Actual amount /g | Actual amount / weight % |
|---|---|---|
| DMABE | 0.5002 | 1.00 |
| BHT | 0.2500 | 0.50 |
| CQ | 0.2503 | 0.50 |
| TOCOPHEROL | | 0.00 |
| EBPADMA | 49.01 | 96.00 |
| Total | | 100.00 |

**Table 3. Composition of monomer mixture B containing no inhibitor.**

| Compound | Actual amount /g | Actual amount / weight% |
|---|---|---|
| DMABE | 0.5002 | 1.00 |
| BHT | | 0.00 |
| CQ | 0.2501 | 0.50 |
| TOCOPHEROL | | 0.00 |
| EBPADMA | 40.252 | 98.50 |
| Total | | 100.00 |

**Table 4. Composition of monomer mixture C containing alpha-Tocopherol.**

| Compound | Actual amount /g | Actual amount / weight % |
|---|---|---|
| DMABE | 0.500 | 1.00 |
| BHT | | 0.00 |
| CQ | 0.2502 | 0.50 |
| TOCOPHEROL | 0.2501 | 0.50 |
| EBPADMA | 49.02 | 96.00 |
| Total | | 100.00 |

To each of the three liquids A, B, or C (25.5 wt%) was separately added 1.1 wt% Aerosil R972 and 73.4 wt% silanized glass filler and the mixtures were mixed (Hauschild SpeedMixer) three times at 1300 rpm for 2 min. Afterwards, they were transferred to a kneeder (IKA Planetron Vertical Kneeder) and kneeded for 10 min at 40°C at 40% of the maximum speed. They were subsequently kneeded four times for 30 min at 80% speed and finally 10 min at 40% speed and 200 mbar.

**Table 5. Composition of a paste containing either of the monomer mixtures A, B, or C.**

| Compound | Actual amount /g | Actual amount / weight % |
|---|---|---|
| TPH3 SPECTRUM GLASS MIXTURE | 110.12 | 73.40 |
| AEROSIL R972 | 1.66 | 1.10 |
| EBPADMA ADDITIVE | 38.36 | 25.50 |
| Total | | 100.00 |

The resulting pastes were inserted into compules with 1.5 mm diameter, capped and stored at indicated temperatures (70°C or 50°C) in an oven until the measurement.

Before the measurement, the compules were kept at 23°C for 2h. Extrusion force was measured at a universal testing machine (ZwickRoell). The compules were emptied by a penetrating needle with 28mm/min. The force is measured using a 500 N force cell. After an initial peak, the plateau-value is noted as extrusion force. An average is calculated from 6 compules.

**The results of the extrusion force measurement of the above pastes are depicted in the** **Fig. 1** **(at the storage temperature of 70 °C) and** **Fig. 2** **(at the storage temperature of 50°C).**

### Example 2: Viscosity of monomer mixtures

Three monomer mixtures comprising 87.63 wt% EBPADMA, 9.74 wt% Ormosil, 0.9 wt% DMABE, 0.24 wt% CQ, 0.74 wt% DPI and either inhibitor of BHT, alpha-Tocopherol or none (0.75 wt%, in the latter case replaced by 0.75 wt% more EBPADMA and Ormosil) were prepared and stirred in the dark at 40°C until all compounds are homogeneously mixed.

**Table 6. Composition of monomer mixture D containing BHT.**

| Compound | Actual amount / weight % | Actual amount / weight g |
|---|---|---|
| EBPADMA Resin: | 87.63 | 21.91 |
| Ormosil: | 9.74 | 2.44 |
| Dimethyl DPI: | 0.74 | 0.19 |
| BHT: | 0.75 | 0.19 |
| CQ: | 0.24 | 0.06 |
| DMABE: | 0.9 | 0.23 |

**Table 7. Composition of monomer mixture E containing alpha-Tocopherol.**

| Compound | Actual amount / weight % | Actual amount / weight g |
|---|---|---|
| EBPADMA Resin: | 87.63 | 21.91 |
| Ormosil: | 9.74 | 2.44 |
| Dimethyl DPI: | 0.74 | 0.19 |
| Tocopherol | 0.75 | 0.19 |
| CQ: | 0.24 | 0.06 |
| DMABE: | 0.90 | 0.23 |

**Table 8. Composition of monomer mixture F containing no inhibitor.**

| Compound | Actual amount / weight % | Actual amount / weight g |
|---|---|---|
| EBPADMA Resin: | 88.30 | 22.08 |
| Ormosil: | 9.82 | 2.45 |
| Dimethyl DPI: | 0.74 | 0.19 |
| CQ: | 0.24 | 0.06 |
| DMABE: | 0.90 | 0.23 |

Ormosil is a mixture from Ormocer, TEGDMA and BisEMA.

The resulting liquids were stored at 70°C in an oven until the measurement (see Figure 3).

Viscosity was measured using a Physica MCR 300 rheometer (CP 1/50, 125 s creep time, 25 data points every 5 s, 10 Pa shear stress) at 23°C and with a waiting time of 5 min.

**Table 9. Detailed parameters for the measurement of the viscosity.**

| | |
|---|---|
| Method | PV-011 |
| Measuring system | CP 1/50 |
| Volume of the sample | ca 0.6 ml |
| Creep time | 125 s |
| Number of data points | 25 |
| Interval of measuring | 5 s (constant) |
| Shear stress | 10 Pa |
| Temperature | 23°C |
| Waiting time | 5 min |

**The results of the viscosity measurement are depicted in the** **Fig. 3****.**

### Example 3: Stability against ambient light of curable dental compositions with different amounts of the at least one polymerization initiator and the at least one polymerization inhibitor

A monomer mixture from 99.75 wt% UDMA-Resin and 0.25 wt% camphorquinone was prepared and stirred in the dark at 50°C until the complete dissolution. The mixture was splitted in two parts and two different concentrations (1 wt% and 0.5 wt%, respectively) of DMABE were added. The mixtures were stirred at 50°C until complete dissolution. Each of the mixtures was splitted in two parts and in every second one, 0.75 wt% dimethyl diphenyl iodonium hexafluorophosphate (DPI) was added. The mixtures were stirred at 50°C until complete dissolution. All four mixtures were each splitted in four parts. Two different concentrations of alpha-Tocopherol or BHT (0.25 wt% and 0.75 wt%, respectively) were added. The 16 mixtures (Fig. 5 (Table 1)) were mixed at 2350 rpm for 6 min (Hauschild SpeedMixer).

Stability against ambient light or alternatively curing or working time was determined using a Suntester CPS. A droplet of the liquid was squeezed between two microscopy slides and put underneath a neutral light filter (ND, D 1,0). The light intensity was adjusted to 9000 Lux. The sample was irradiated in steps of 10s and checked if the slides can be slided against each other until polymerization.

The accumulated time was noted. The average time of multiple experiments was used as curing time.

**The results of the above measurements are depicted in** **Fig. 5** **(Table 1).**

### Example 4: Stability against ambient light of curable dental compositions comprising isomers of Tocopherol and its ester

Five mixtures comprising 98.75 wt% EBPADMA, 0.75 wt% DMABE, 0.25 wt% CQ, and either inhibitor of BHT, alpha-Tocopherol, Delta-Tocopherol and D-Alpha-Tocopherol succinate or none (0.25 wt%, in the latter case replaced by 0.25 wt% more EBPADMA) were prepared and stirred for in the dark at 40°C until all compounds are homogeneously mixed.

Stability against ambient light or alternatively curing or working time was determined using a Suntester CPS. A droplet of the liquid was squeezed between two microscopy slides and put underneath a neutral light filter (ND, D 1,0). The light intensity was adjusted to 9000 Lux. The sample was irradiated in steps of 10 s and checked if the slides can be slided against each other until polymerization. The accumulated time was noted. The average time of multiple experiments was used as curing time, in accordance with ISO4049.

**The results of the above measurements are depicted in** **Fig. 4****.**

## Claims

1. A curable dental composition comprising a monomer mixture and optionally at last one pigment, wherein the monomer mixture comprises at least one polymerizable monomer, at least one polymerization initiator and at least one polymerization inhibitor, wherein the at least one polymerization inhibitor is Tocopherol, its derivative, ether or ester or one of their isomers or a mixture thereof.

2. The curable dental composition of claim 1, wherein the curable dental composition is a light curable dental composition.

3. The curable dental composition of claim 2, wherein the curable dental composition comprises a photoinitiator.

4. The curable dental composition of claims 1-3, wherein the curable dental composition comprises additionally at least one filler.

5. The curable dental composition of claims 1-4, wherein the curable dental composition comprises one or more compounds selected from a group comprising a UV stabilizer, a rheological modifier, a fluorescent agent, an acidic component, a chain transfer agent, an X-ray opaquer, a silanization agent, a reactive glass and a solvent.

6. The curable dental composition of claims 1-5, wherein the at least one polymerizable monomer is selected from the group comprising a monofunctional or oligofunctional or polyfunctional vinyl, or acrylate or a methacrylate including but not limited to methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate, hydroxypropyl acrylate, hydroxypropyl methacrylate, tetrahydrofurfuryl acrylate, tetrahydrofurfuryl methacrylate, glycidyl acrylate, glycidyl methacrylate, the diglycidyl methacrylate of bis-phenol A, glycerol mono-and diacrylate, glycerol mono- and di- methacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate, neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethylhexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1 -chloromethyl-2-methacryloxy ethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane, 2,2'-bis[3(4-phenoxy)-2-hydroxypropane-1-acryalte]propane, N,N'-diallyl-1,4- bisacrylamido-(2E)-but-2-ene (BAABE) and N,N'-diethyl-1,3-bisacrylamido-propane (BADEP).

7. The curable dental composition according to any of the preceding claims, wherein the at least one polymerizable monomer is selected from the group comprising EBPADMA-Resin, UDMA-Resin, TEGDMA and BisEMA.

8. The curable dental composition according to any of the preceding claims, wherein the at least one polymerization initiator is selected from the group comprising a visible light and an ultraviolet light photoinitiator including an alpha-diketone with or without at least one additional hydrogen donor.

9. The curable dental composition according to any of the preceding claims, wherein the at least one polymerization initiator is selected from the group comprising CQ, DMABE and DPI.

10. The curable dental composition according to any of the preceding claims, wherein the at least one filler is selected from the group comprising a glass filler, a silanated glass filler, zirconium oxide, fluorides, an organic filler, a hybrid organic/inorganic filler and a combination thereof.

11. The curable dental composition according to claims 10, wherein the at least one filler is a silanated glass filler.

12. The curable dental composition according to any of the preceding claims, wherein the at least one pigment is selected from the group comprising iron oxide, titanium oxide, barium sulfate, manganese oxide, zinc oxide, bismuth oxide and organic fluorophores.

13. The curable dental composition according to any of the preceding claims, wherein the concentration of the at least one polymerization inhibitor in the curable dental composition is from 0.001wt% to 3 wt%.

14. The curable dental composition according to any of the preceding claims, wherein the concentration of the at least one polymerization inhibitor in the curable dental composition is from 0.005wt% to 1 wt%.

15. The curable dental composition according to any of the preceding claims, wherein the concentration of the at least one polymerization inhibitor in the curable dental composition is from 0. 01wt% to 0.75 wt%.

16. The curable dental composition according to any of the preceding claims for producing a composite, a temporary sealing material, a dental cement, an adhesive or a fissure sealant.

17. The curable dental composition according to any of the preceding claims for use in the dental field.
